# EUROPÄISCHE PATENTANMELDUNG

(11) **EP 3 244 206 A1**
(43) Veröffentlichungstag der Anmeldung: **15.11.2017**
(21) Anmeldenummer: 16001089.8
(22) Anmeldetag: 13.05.2016
(51) Int. Cl.: G01N 33/00

(54) **KODIERUNGSKOMPONENTE ZUM KODIEREN VON GASEN SOWIE EIN ENTSPRECHEND KODIERTES GAS**

(71) Anmelder: Linde Aktiengesellschaft, 80331 München (DE)
(72) Erfinder: Fieret, Jim, Bell Hill, Hook Norton OX15 5PS (GB); Trautmann, Andreas, 80805 München (DE)
(74) Vertreter: Gellner, Bernd

(57) **Zusammenfassung**

Gasförmige Kodierungskomponente zum Kodieren von Gasen, wobei die gasförmige Kodierungskomponente ein oder mehrere Isotope eines Gases umfasst und der Anteil des zumindest einen Isotops gegenüber dem natürlich vorkommenden Anteil dieses Isotops im Gas verändert ist.

## Beschreibung

Hersteller von technischen Gasen bzw. Spezialgasen stellen relativ teure und exakt gemischte Gasmischungen für breite Anwendungsgebiete, wie z.B. CO₂-Laserresonatoren oder Kalibrierungsmischungen usw., her. Derartige Gasmischungen werden in Gasbehältern abgefüllt, die mit der exakten Gaszusammensetzung der Gasmischung etikettiert sind.

Kunden können beim Hersteller reklamieren, wenn die Gaszusammensetzung außerhalb der auf dem Etikett angegebenen Bereiche bzw. Werte für die Gaszusammensetzung liegt. Dann analysiert der Gashersteller oder eine unabhängige dritte Partei die Gasmischung um festzustellen, ob die im Behälter bevorratete Gasmischung mit der angegebenen Spezifizierung übereinstimmt.

Wenn die Gaszusammensetzung nicht der in der Spezifikation angegebenen Gaszusammensetzung entspricht, übernimmt der Gashersteller die Verantwortung. Sollte die Gaszusammensetzung der Spezifikation entsprechen, weiß der Kunde in der Regel, dass seine Probleme mit der Gasmischung andere Ursachen haben.

Einige Kunden wollen beispielsweise keine hochwertigen Gasmischungen von angesehenen Gasherstellern kaufen. Sie verwenden daher leere Gasbehälter, die immer noch mit dem Logo und dem Namen sowie der Gaszusammensetzung des Originalherstellers etikettiert sind, wobei die Etikettierung in der Regel neben der Gaszusammensetzung auch Reinheitszertifikate umfasst, und bringen diese zu Gasfüllfirmen und lassen die Originalgasmischung durch ein niedrigpreisiges Ersatzgas ersetzen.

Mit diesen Ersatzgasen kommt es regelmäßig zu Problemen aufgrund von Verunreinigungen oder dadurch, dass die Gaszusammensetzung nicht exakt der gewünschten Zusammensetzung bzw. der Zusammensetzung, mit der der Gasbehälter etikettiert ist, entspricht. Wenn die Kunden sich dann beim Originalhersteller des Gaszylinders beschweren und die Erstbefüllung vom Gashersteller oder einem autorisierten Dritthersteller erfolgte, hat der Originalhersteller keinerlei Möglichkeiten zu beweisen, dass er den Gasbehälter nicht befüllt hat, sondern dass der Gasbehälter von einem Dritten befüllt wurde.

Aufgabe der vorliegenden Erfindung ist es, eine Möglichkeit zum Markieren bzw. Kodieren von Gaszusammensetzungen bereitzustellen, so dass es auf einfache Art und Weise möglich ist zu identifizieren, ob eine in einem Gasbehälter bevorratete Gasmischung von einer autorisierten Befüllstation oder von einem unautorisierten Dritten befüllt wurde.

Erfindungsgemäß ist eine Kodierungskomponente zum Kodieren von Gasen vorgesehen. Diese zeichnet sich dadurch aus, dass die Kodierungskomponente ein oder mehrere Isotope eines Gases umfasst, wobei der Anteil des zumindest einen Isotops gegenüber dem natürlich vorkommenden Anteil dieses Isotops im Gas verändert ist.

Mit der erfindungsgemäßen gasförmigen Kodierungskomponente ist es erstmals möglich zu identifizieren, ob ein Gasbehälter mit einer Gasmischung von einer autorisierten Befüllstation befüllt wurde oder ob die Befüllung durch einen unautorisierten Dritten erfolgt ist.

Gasförmige Isotope sind Spezialprodukte und können weltweit nur von einigen Anbietern bezogen werden. Dieser Umstand in Verbindung mit den komplexen Analyseverfahren bzw. Analysevorrichtungen macht die gasförmigen Isotope ideal als Marker für Gasmischungen, wobei der Gashersteller ohne es jemanden mitzuteilen, den Anteil eines oder mehrerer Isotope erhöht oder verringert, so dass sich die Konzentrationen einzelner Isotope von ihren natürlich vorkommenden Verhältnis unterscheidet.

Somit ist es möglich einen Gasbehälter zu testen und dann festzustellen, ob dieser von einem autorisierten Anbieter befüllt wurde oder nicht. Je nachdem, ob die Isotopenkonzentration dem vom Hersteller vorgegebenen Verhältnis entspricht oder nicht.

Somit ist es erfindungsgemäß möglich, die Verwendung von nicht autorisierten Gasmischungen bzw. Gasmischungen, beispielweise für Kohlenstoffdioxid-Laserresonator-Gasmischungen oder von gasförmigen Kalibriermischungen von Dritten nachzuweisen.

Das erfindungsgemäße Verfahren kann auf alle gasförmigen, flüssigen oder festen Produkte der Industrie zu jeglichem Zweck angewendet werden.

Die Häufigkeit des Isotops kann gegenüber der natürlich vorkommenden Häufigkeit um mehr als 0,5% oder um mehr als 1,0% oder um mehr als 1,5% oder um mehr als 2,5% oder um mehr als 5,0% oder um mehr als 10,0% oder um mehr als 25% oder um mehr als 50,0% oder um mehr als 75,0 % oder um mehr als 100% oder um mehr als 150% oder um mehr als 200% oder um mehr als 500% oder um mehr als 1000% erhöht oder verringert sein.

Die Kodierungskomponente kann zumindest ein Isotop eines Aktivgases umfassen.

Die Kodierungskomponente kann zumindest ein Isotop eines inerten Gases umfassen.

Die Kodierungskomponente kann mehrere unterschiedliche Isotope in vorbestimmten Verhältnissen enthalten, wobei diese Verhältnisse die Kodierung ausbilden.

Weiterhin kann erfindungsgemäß ein kodiertes Gas vorgesehen sein. Dieses umfasst ein Prozessgas und zeichnet sich dadurch aus, dass das Prozessgas eine Kodierungskomponente enthält.

Zudem kann erfindungsgemäß eine Kodierungskomponente verwendet werden, um ein Gas zu kodieren, so dass dieses eindeutig identifizierbar ist.

Ein Beispiel für ein unübliches stabiles Isotop, welches die Kodierungskomponente ausbilden kann, ist beispielsweise Stickstoff-15, das normalerweise zu 0,364% in reinem natürlichem Stickstoff enthalten ist, wobei der restliche Anteil Stickstoff-14 ist.

Ein weiteres Isotop kann Kohlenstoff-13-Dioxidgas sein, das in etwa 1% in natürlichem Kohlendioxidgas vorkommt. Den Rest bildet Kohlenstoff-12-Dioxid.

Ein weiteres Beispiel für stabile Isotope können Sauerstoff-17 und Sauerstoff-18. Isotope sein, die 0,037% und 0,204% in reinem Sauerstoff enthalten sind, wobei der Rest Sauerstoff-16-Isotope sind.

Es gibt weitere chemische gasförmige Elemente, die einige ungewöhnliche stabile Isotope haben, wie z. B. Xenon, Neon, Krypton, Chlor und Fluor, die ebenfalls in der Kodierungskomponente enthalten sein können.

Es ist nicht möglich, verschiedene stabile Isotope mittels chemischer Analyseverfahren zu detektieren, da sie sich hinsichtlich ihrer Chemie annähernd gleich verhalten. Stabile Isotope haben nahezu ein identisches chemisches Verhalten, so dass eine Veränderung in ihrer entsprechenden Konzentration die Verwendung nicht beeinflusst, wie z.B. bei CO₂-Lasern oder bei einer Kalibrierungseinheit für Abgassensoren in der Automobilindustrie.

Die erfindungsgemäß veränderten Isotopenverhältnisse in einer Kodierungskomponente können nur durch komplexe Analysetechniken, beispielsweise mittels eines Massenspektrometers, identifiziert bzw. detektiert werden.

Es ist auch möglich, unübliche stabile Isotope in Verbundmolekülen zu kombinieren, wie z. B. Carbon-Kohlenstoffdioxid, Kohlenmonoxid, bei denen eine oder mehrere atomare Bestandteile der Moleküle ungewöhnliche stabile Isotope sind.

Das Prozessgas kann ein inertes Gas, wie z.B. Argon, Helium, Neon, Krypton, Xenon, Radon oder auch Mischungen daraus, und/oder ein Aktivgas, wie z.B. O₂, CO₂, H₂, und N₂, oder auch Mischungen daraus, umfassen.

Eine Mischung aus Prozessgas und Kodierungskomponente wird im Folgenden als kodiertes Gas bezeichnet. Als Kodierungskomponente, die mit einem entsprechenden Prozessgas vermischt oder auch in reiner Form verwendet werden kann, ist vorzugsweise Sauerstoff 18 Kohlendioxid (C¹⁸O₂), Kohlenstoff 13 Kohlendioxid (¹³CO₂), Kohlenstoff 13 Kohlenmonoxid (¹³CO₂), Deuterium (D₂), Stickstoff 15 (¹⁵N₂) und Sauerstoff 18 (¹⁸O₂) vorgesehen.

Die Kodierungskomponente umfasst beispielsweise ein oder mehrere Isotope eines Gases, vorzugsweise eines Gases einer entsprechenden in einem Gasbehälter enthaltenen Gasmischung, wobei der Anteil eines Isotops gegenüber dem natürlichen Anteil der Isotope im Gas verändert ist. Das bedeutet das Verhältnis der Isotope ist gegenüber dem natürlich vorkommenden Verhältnis verändert. Beispielsweise bei Stickstoff das Verhältnis von ¹⁴N (Häufigkeit = 99,634) zu ¹⁵N (Häufigkeit = 0,366) derart verändert, dass der Anteil an ¹⁵N erhöht und der Anteil an ¹⁴N verringert ist oder umgekehrt. Beispielsweise bei Kohlenstoff das Verhältnis von ¹²C (Häufigkeit = 98,9) zu ¹³C (Häufigkeit = 1,1) derart verändert, dass der Anteil an ¹³C erhöht und der Anteil an ¹²C verringert ist oder umgekehrt. Beispielsweise kann bei Wasserstoff das Verhältnis von ¹H (Häufigkeit = 98,9885) zu ²H (Häufigkeit = 0,0115) derart verändert werden, dass der Anteil an ²H erhöht und der Anteil an ¹H verringert ist oder umgekehrt.

Als Isotope sind vorzugsweise Stickstoff-15 und Stickstoff-14 und/oder Kohlenstoff-12, Kohlenstoff-13 und/oder Kohlenstoff-14 und/oder auch beispielsweise Sauerstoff-16 und/oder Sauerstoff-18 vorgesehen. Weiterhin kann auch Argon -36, -38,-39, -40 vorgesehen sein.

Grundsätzlich denkbar ist auch die Verwendung von Wasserstoff-2- oder Wasserstoff-3-sowie Helium-3- und Helium-4-Isotopen.

Um komplexere Kodierungen vorzusehen, können auch zwei oder drei oder mehr verschiedene Isotope in der Kodierungskomponente enthalten sein. Demgemäß kann die Kodierungskomponente ein oder mehrere andere als die natürlich vorkommenden Isotope eines oder mehrere Gase umfassen. Bspw. können Sauerstoff-Isotope mit Stickstoff-Isotopen oder auch C-Isotope im CO₂ mit H-Isotopen in H₂ kombiniert werden.

Bei der Verwendung einer Kodierungskomponente kann eine Datenbank zum Abspeichern von Kodierungsinformationen bzw. den entsprechend veränderten Isotopenverhältnissen vorgesehen sein.

Das Prozessgas kann ein inertes Gas, wie z.B. Argon, Helium, Neon, Krypton, Xenon oder Radon und/oder ein Aktivgas, wie z.B. O₂, CO₂, H₂, und N₂ oder auch Mischungen daraus umfassen.

Stickstoff 15N-Isotope können sich abhängig vom Legierungselement, der Temperatur, der Konzentration und/ oder der Reaktionszeit manchmal inert und manchmal reaktiv abhängig vom Legierungselement, Temperatur, Konzentration und Reaktionszeit verhalten.

Kohlenstoff-Isotope ¹²C und ¹³C werden in Form von Kohlendioxid bereitgestellt, welches dann im Verfahren abgetrennt wird.

Die Isotope der Kodierungskomponente können Isotope des Prozessgases sein.

Die Isotope der Kodierungskomponente können auch verschieden zu den Isotopen des Prozessgases sein.

Beispiele für genaue Prozentangaben zur Erhöhung oder Verringerung der Isotopenverhältnisse in der Kodierungskomponente sind in der nachfolgenden Tabelle angegeben.

| Art der Kodierung | | | | | |
|---|---|---|---|---|---|
| | Element | Art des Isotops das zum Anreichern eines Basisgases verwendet wird um eine Codierung vorzusehen | Natürlich vorkommende Konzentration der Isotope | Mögliche Moleküle | Bereich der Isotopen Zudosierung zu einem Basisgas |
| Inerte Isotope | Ar | ³⁶Ar | ³⁶Ar: 0.337% | N/A | Zwischen dem 1.1-fachen und dem 10-fachen des natürlich vorkommenden Anteil des Isotops oder kleiner gleich dem 0.9-fachen des natürlichen Anteils |
| | | | ³⁸Ar: 0.063% | | |
| | | | ⁴⁰Ar: 99.6% | | |
| | He | ³He | ³He: 0.000137% Rest: ⁴He | N/A | Zwischen dem 1.1-fachen und dem 10-fachen des natürlich vorkommenden Anteil des Isotops oder kleiner gleich dem 0.9-fachen des natürlichen Anteils |
| | H | ²H | ²H: 0.012% Rest ¹H | ²H₂ | ²H₂: Zwischen 1 ppm und 10 ppm |
| | | | | ²H¹H | ²H¹H: Zwischen dem 1.1-fachen und dem 10-fachen des natürlich vorkommenden Anteil des Isotops oder kleiner gleich dem 0.9-fachen des natürlichen Anteils N²H₃ : Zwischen 1 ppm und 10 ppm |
| | | | | N²H₃ | |
| | Kr | ⁷⁸Kr | ⁷⁸Kr: 0.35% | N/A | ⁷⁸Kr und ⁸²Kr: Zwischen dem 1,1-fachen und dem 10-fachen des natürlich vorkommenden Anteil des Isotops oder kleiner gleich dem 0.9-fachen des natürlichen Anteils. |
| | | ⁸²Kr | ⁸⁰Kr: 2.25% | | |
| | | ⁸⁴Kr | ⁸²Kr:11.6% | | |
| | | ⁸⁶Kr | ⁸³Kr: 11.5% | | |
| | | | ⁸⁴Kr: 17.3% | | Andere: Zwischen dem 1,001-fachen und dem 1,1-fachen des natürlich vorkommenden Anteil des Isotops oder kleiner gleich dem 0.99-fachen des natürlichen Anteils |
| | | | ⁸⁶Kr: 17.3% | | |
| | Ne | ²⁰Ne²¹Ne²²Ne | ²⁰Ne: 90.48% | N/A | ²¹Ne und ²²Ne: Zwischen dem 1,001-fachen und dem 1,1-fachen des natürlich vorkommenden Anteil des Isotops oder kleiner gleich dem 0.99-fachen des natürlichen Anteils |
| | | | ²¹Ne: 0.27% | | |
| | | | ²²Ne: 9.25% | | |
| | Xe | ¹²⁴Xe | ¹²⁴Xe: 0.095% | N/A | ¹²⁴Xe, ¹²⁹Xe: Zwischen dem 1,1-fachen und dem |
| | | ¹²⁹Xe | ¹²⁶Xe: 0.089% | | 10-fachen des natürlich vorkommenden Anteil |
| | | ¹³¹Xe | ¹²⁸Xe: 1.91% | | des Isotops oder kleiner gleich dem 0.9-fachen |
| | | ¹³²Xe | ¹²⁹Xe: 26.4% | | des natürlichen Anteils. |
| | | ¹³⁴Xe | ¹³⁰Xe: 4.07% | | Andere: Zwischen dem 1,001-fachen und dem |
| | | ¹³⁶Xe | ¹³¹Xe: 21.2% | | 1,1-fachen des natürlich vorkommenden Anteil |
| | | | ¹³²Xe: 26.9% | | des Isotops oder kleiner gleich dem 0.99-fachen |
| | | | ¹³⁴Xe: 10.4% | | des natürlichen Anteils |
| | | | ¹³⁶Xe: 8.86% | | |
| Reaktive Isotope, | C | ¹²C | ¹²C: 98.8% | ¹²CO | ¹³CO, ¹³CO₂: Zwischen dem 1,1-fachen und dem 10-fachen des natürlich vorkommenden Anteil des Isotops oder kleiner gleich dem 0.9-fachen des natürlichen Anteils |
| | | ¹³C | ¹³C: 1.1% | ¹³CO ¹³CO₂ | |
| | O | ¹⁷O | ¹⁶O: 99.76% | ¹⁸O₂ | ¹⁷O₂, ¹⁸O₂, C¹⁸O₂: Zwischen dem 1,1-fachen und dem 10-fachen des natürlich vorkommenden Anteil des Isotops oder kleiner gleich dem 0.9-fachen des natürlichen Anteils der beiden Sauerstoff Isotope |
| | | ¹⁸O | ¹⁷O: 0.039% | ¹⁷O₂ | |
| | | | ¹⁸O: 0.201% | C¹⁸O₂ | |
| | N | ¹⁵N | ¹⁴N: 99.634% | ¹⁵N₂ | ¹⁵N₂, ¹⁵NH₃: Zwischen dem 1,01-fachen und dem 1,1-fachen des natürlich vorkommenden Anteil des Isotops oder kleiner gleich dem 0.99-fachen des natürlichen Anteils des ¹⁵N Isotops |
| | | | ¹⁵N: 0.366% | ¹⁵NH₃ | |

## Patentansprüche

1. Gasförmige Kodierungskomponente zum Kodieren von Gasen,
**dadurch gekennzeichnet,**
**dass** die gasförmige Kodierungskomponente ein oder mehrere Isotope eines Gases umfasst, wobei der Anteil des zumindest einen Isotops gegenüber dem natürlich vorkommenden Anteil dieses Isotops im Gas verändert ist.

2. Kodierungskomponente nach Anspruch 1,
**dadurch gekennzeichnet,**
**dass** die Häufigkeit des Isotops gegenüber der natürlich vorkommenden Häufigkeit um mehr als 0,5% oder um mehr als 1,0% oder um mehr als 1,5% oder um mehr als 2,5% oder um mehr als 5,0% oder um mehr als 10,0% oder um mehr als 25% oder um mehr als 50,0% oder um mehr als 75% oder um mehr als 100% oder um mehr als 150% oder um mehr als 200% oder um mehr als 500% oder um mehr als 1000% erhöht oder verringert ist.

3. Kodierungskomponente nach Anspruch 1 oder 2,
**dadurch gekennzeichnet,**
**dass** die Kodierungskomponente zumindest ein Isotop eines Aktivgases umfasst.

4. Kodierungskomponente nach einem der Ansprüche 1 bis 3,
**dadurch gekennzeichnet,**
**dass** die Kodierungskomponente zumindest ein Isotop eines inerten Gases umfasst.

5. Kodierungskomponente nach einem der Ansprüche 1 bis 4,
**dadurch gekennzeichnet,**
**dass** die Kodierungskomponente mehrere unterschiedliche Isotope unterschiedlicher Gase und/oder ein und desselben Gases in vorbestimmten Verhältnissen enthält, wobei diese Verhältnisse die Kodierung ausbilden.

6. Kodiertes Gas umfassend
ein Prozessgas
**dadurch gekennzeichnet,**
**dass** das Prozessgas eine Kodierungskomponente gemäß einem der Ansprüche 1 bis 5 enthält.

7. Kodiertes Gas nach Anspruch 6,
**dadurch gekennzeichnet,**
**dass** die Isotope der Kodierungskomponente Isotope des Prozessgases sind.

8. Kodiertes Gas nach Anspruch 6 oder 7,
**dadurch gekennzeichnet,**
**dass** die Isotope der Kodierungskomponente verschieden zu den Isotopen des Prozessgases sind.

9. Kodiertes Gas nach Anspruch 8,
**dadurch gekennzeichnet,**
**dass** das Prozessgas ein inertes Gas, wie z.B. Argon, Helium, Neon, Krypton, Xenon oder Radon oder ein Aktivgas, wie z.B. O₂, CO₂, H₂, und N₂ oder auch Mischungen daraus umfasst und die Kodierungskomponente Sauerstoff 18 Kohlendioxid (C¹⁸O₂), Kohlenstoff 13 Kohlendioxid (¹³CO₂), Kohlenstoff 13 Kohlenmonoxid (¹³CO₂), Deuterium (D₂), Stickstoff 15 (¹⁵N₂) und Sauerstoff 18 (¹⁸O₂) oder auch Mischungen daraus umfasst.

10. Verwendung einer Kodierungskomponente gemäß einem der Ansprüche 1 bis 5 zum Kodieren von Gasen.
